(19) Europäisches Patentamt
European Patent Office
Office européen des brevets

(11) Publication number: 0 477 678 A1

# (12) EUROPEAN PATENT APPLICATION

(21) Application number: 91115432.6

(22) Date of filing: 12.09.91

(51) Int. Cl.[5]: C07D 213/78, C07D 213/83, A01N 47/40

(30) Priority: 25.09.90 JP 251840/90

(43) Date of publication of application: 01.04.92 Bulletin 92/14

(84) Designated Contracting States: BE CH DE FR GB IT LI NL

(71) Applicant: NIHON BAYER AGROCHEM K.K.
7-1, Nihonbashi Honcho 2-chome Chuo-ku
Tokyo 103(JP)

(72) Inventor: Kurahashi, Yoshio
47-15, Oya-machi
Hachioji-shi, Tokyo(JP)
Inventor: Shiokawa, Kozo
2-23-30, Shukugawara, Tama-ku
Kawasaki-shi, Kanagawa(JP)
Inventor: Moriya, Koichi
3-19-5, Ekiminami-machi
Oyama-shi, Tochigi(JP)
Inventor: Shibuya, Katsuhiko
1-9-31, Wakagi-cho
Oyama-shi, Tochigi(JP)
Inventor: Izumi, Tatsuji
3-20-45, Wakagi-cho
Oyama-shi, Tochigi(JP)
Inventor: Sakuma, Haruhiko
3-12-15, Ekiminami-machi
Oyama-shi, Tochigi(JP)

(74) Representative: Schumacher, Günter, Dr. et al
c/o Bayer AG Konzernverwaltung RP
Patentabteilung
W-5090 Leverkusen 1 Bayerwerk(DE)

## (54) O-Acyl formaldoxime-pyridines, process for their preparation and their use as microbicides.

(57) O-Acyl-formaldoxime pyridines of the formula (I)

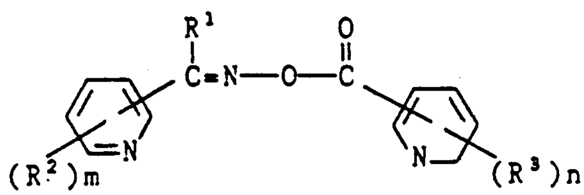

(I)

in which
$R^1$ is halogen or $C_{1-4}$ alkylthio,
$R^2$ is halogen or $C_{1-4}$ alkyl,
$R^3$ is halogen, $C_{1-4}$ alkyl, $C_{1-4}$ alkoxy,
$C_{1-4}$ alkylthio, $C_{1-2}$ alkylamino,
$C_{2-4}$ (in total) dialkylamino or halo-$C_{1-2}$ alkyl,
m is 0 or 1, and
n is 0, 1 or 2
and their use as microbicides.

EP 0 477 678 A1

The present invention relates to novel O-acyl formaldoxime-pyridines, to processes for their preparation and their use as microbicides, especially agricultural fungicides.

It has already been disclosed that O-acyl (α-nitroformaldoxime)- and (α-haloformaldoxime)-pyridines have fungicidal properties in the field of agriculture (see USP 4,244,959).

There have now been found novel O-acyl formaldoxime-pyridines of the formula (I)

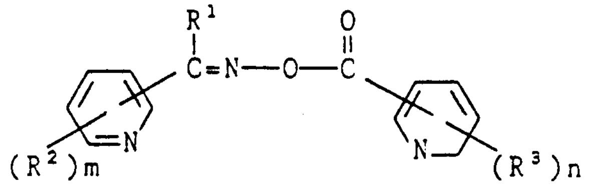

(I)

wherein
$R^1$ is halogen or $C_{1-4}$ alkylthio,
$R^2$ is halogen or $C_{1-4}$ alkyl,
$R^3$ is halogen, $C_{1-4}$ alkyl, $C_{1-4}$ alkoxy,
$C_{1-4}$ alkylthio, $C_{1-2}$ alkylamino,
$C_{2-4}$ (in total) dialkylamino or halo-$C_{1-2}$ alkyl,
m is 0 or 1, and
n is 0, 1 or 2.

The compounds of the formula (I) can be present as geometric isomers (syn/anti) or isomer mixtures of various compositions. Both the pure isomers and the isomer mixtures are subject according to the invention.

The compounds of the formula (I) can be obtained by a process in which

a) compounds of the formula (II)

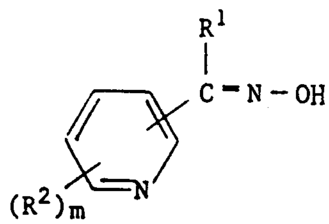

(II)

wherein $R^1$, $R^2$ and m have the same meanings as mentioned above,

are reacted with compounds of the formula (III)

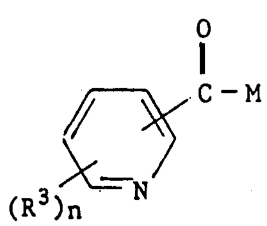

(III)

wherein $R^3$ and n have the same meanings as mentioned above, and

M represents halogen atom,

in the presence of inert solvents, and if appropriate in the presence of an acid binder,

or

b) where $R^1$ represents $C_{1-4}$ alkylthio,

compounds of the formula (Ia)

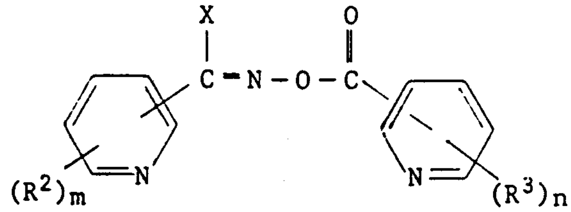

(Ia)

wherein $R^2$, $R^3$, m and n have the same meanings as mentioned above, and
X represents halogen atom,
are reacted with alkylmercaptans of the formula (IV)

alkyl SH (IV)

or their alkali salts, preferably sodium salt,
in the presence of inert solvents and if appropriate in presence of a base.

The O-acyl formaldoxime-pyridines exhibit powerful microbicidal properties.

Surprisingly, the compounds of the formula (I) exhibit interalia, a substantially greater fungicidal action than known compounds of the prior art, close to the present invention.

In the formulae, halogen represents fluorine, chlorine, bromine or iodine, and preferably fluorine, chlorine or bromine, and particularly chlorine.

$C_{1-4}$ alkyl, and the alkyl part in $C_{1-4}$ alkoxy and $C_{1-4}$ alkylthio represent methyl, ethyl, propyl, iso-propyl or n-(iso-, sec- or tert-) butyl, and preferably methyl, ethyl, propyl or iso-propyl, and particularly methyl.

The alkyl part in $C_{1-2}$ alkylamino, halo-$C_{1-2}$ alkyl and $C_{2-4}$ (in total) dialkylamino represents methyl or ethyl, and preferably methyl.

The halogen part in halo-$C_{1-2}$ alkyl represents the same definitions as stated above, and preferably fluorine.

Among the compounds of the formula (I), according to the invention, preferred compounds are those in which
$R^1$ is fluorine, chlorine, bromine or methylthio,
$R^2$ is chlorine or methyl,
$R^3$ is fluorine, chlorine, bromine, methyl, methoxy, methylthio, methylamino, dimethylamino or trifluoromethyl,
m is 0 or 1, and
n is 0, 1 or 2.

Particularly preferred compounds of the formula (I) are those in which
$R^1$ is chlorine or methylthio,
$R^2$ is chlorine or methyl,
$R^3$ is fluorine, chlorine, bromine, methyl, methoxy, methylthio or trifluoromethyl,
m is 0 or 1, and
n is 0, 1 or 2.

All the definitions of $R^1$ to $R^3$, m and n can be combined one with another, for example, the preferred definition of $R^1$ with the very particularly preferred definition of $R^3$ and so on.

When use is made, in the above-mentioned process a), of 3-(α-chloro-formaldoxime)-pyridinium hydrochloride and 6-chloro-nicotinoyl chloride as starting materials, for example, the reaction can be expressed as follows:

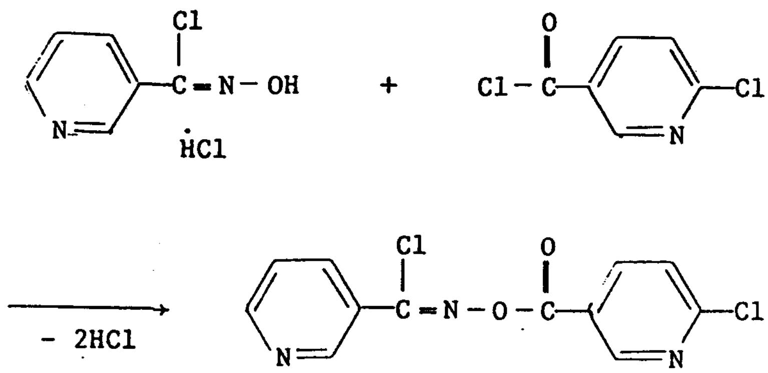


When use is made, in the above-mentioned process b), of O-6-chloronicotinoyl-3-(α-chloro-formaldoxime)-pyridine and sodium methyl-mercaptan as starting materials, the reaction can be expressed as follows:

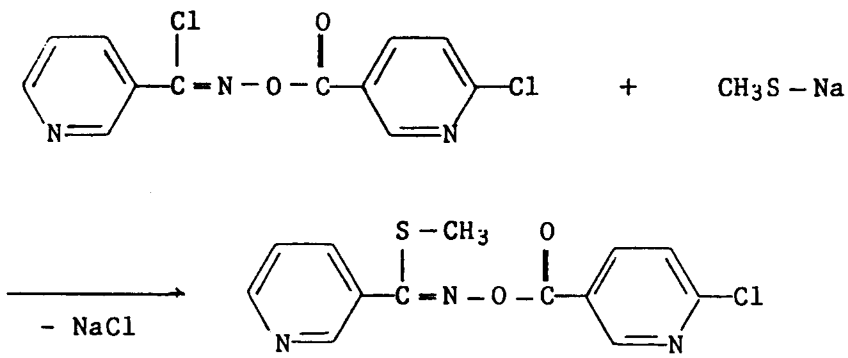


In the process a), the compounds of the formula (II) as a starting material mean ones based on the aforementioned definitions of $R^1$, $R^2$ and m.

In the formula (II), $R^1$, $R^2$ and m preferably have the same meanings as given for the formula (I).

The compounds of the formula (II), where $R^1$ represents halogen, include known compounds described in USP 4,244,959, and in general, according to Organic Synthesis, Coll., vol. V504 (1973), Houben Weyl VII, 691, ibid., x/3, pages 847 and 854, and ibid., x/4, p98, may be obtained when compounds of the formula (V)

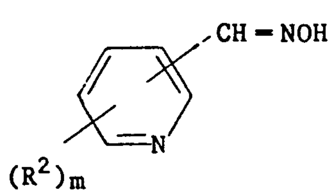

(V)

wherein $R^2$ and m have the same meanings as mentioned above, are reacted with elementary halogen.

Where in the formula (II), $R^1$ represents $C_{1-4}$ alkylthio, said compounds of the formula (II) may be obtained, for example, when compounds of the formula (II-1)

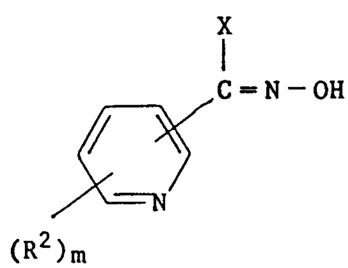

(II-1)

wherein $R^2$, m and X have the same meanings as mentioned above, are reacted with the aforementioned compounds of the formula (IV).

As examples of the compounds of the formula (II), there may be mentioned:
3-(α-chloro-formaldoxime)-pyridinium hydrochloride,
2-(α-methylthio-formaldoxime)-pyridine,
4-(α-chloro-formaldoxime)-pyridinium hydrochloride, and
2-(α-chloro-formaldoxime)-pyridinium hydrochloride.

In the process a), the compounds of the formula (III) as also a starting material mean ones based on the aforementioned $R^3$, n and M.

In the formula (III), $R^3$ and n preferably have the same meanings as given for the formula (I), and M represents halogen preferrably chlorine.

The compounds of the formula (III) are well-known in the field of organic chemistry.

As examples, there may be mentioned:
6-chloronicotinoyl chloride,
5,6-dichloronicotinoyl chloride,
4-(2,6-dichloro)-pyridyl carbonyl chloride,
6-fluoronicotinoyl chloride,
5-bromonicotinoyl chloride,
2-methylthionicotinoyl chloride,
5-chloronitocinoyl chloride and the like.

In the process b), the compounds of the formula (Ia) as a starting material are a part of the compounds of the formula (I), according to the invention, which can be prepared by the process a).

The compounds of the formula (IV) are well-known and as an example thereof, sodium methylmercaptane is exemplified.

In carrying out the above-mentioned process a), it is possible to use any inert organic solvents as the suitable diluents. Examples of the diluents are: water; aliphatic, cycloaliphatic and aromatic hydrocarbons which may be chlorinated hydrocarbons, such as hexane, cyclohexane, petroleum ether, ligroin, benzene, toluene, xylene, methylene chloride, chloroform, carbon tetrachloride, ethylene chloride, chlorobenzene, and the like; ethers such as diethyl ether, methyl ethyl ether, di-isopropyl ether, dibutyl ether, dioxane, tetrahydrofuran and the like; ketones such as acetone, methyl ethyl ketone, methyl isopropyl ketone, methyl isobutyl ketone, etc.; nitriles such as acetonitrile, propionitrile, acrylonitrile and the like; esters such as ethyl acetate, amyl acetate, etc.; acid amides such as dimethyl formamide, dimethyl acetamide and the like; sulfones and sulfoxides such as dimethyl sulfoxide, sulfolane and the like; and bases such as pyridine, etc.

In carrying out the above-mentioned process a), use may be made of an acid binder selected from the group consisting, for example, of hydroxides, carbonates, bicarbonates and alcoholates of alkali metals; and tertiary amines such as, for example, triethylamine, diethylaniline, pyridine, etc.

In carrying out the above-mentioned process a), the reaction temperature can be varied within a substantially wide range. For instance, the reaction may be effected at a temperature between about -20 °C and about +120 °C, preferably at a temperature of from about 0 °C to about 30 °C. It is preferred to carry out the reaction under normal pressure, although the use of a higher or lower pressure is also allowable.

In carrying out the reaction of the above-mentioned process a), use is made, for example, of the compounds of the formula (III) in a mole amount from 1.0 to 1.1 moles per one mole of the compounds of the formula (II) in the presence of an inert solvent such as, for example, methylene chloride and, if necessary, an acid binder to obtain the desired compounds of the formula (I).

In carrying out the above-mentioned process b), it is possible to use any inert organic solvents as the suitable diluents. Examples of the diluents are those mentioned in the case of the process a).

In the process b), the reaction temperature may be varied within a substantially wide range. The process b) may generally be carried out at a temperature of about -20 °C to +120 °C, preferably a temperature of about 30 to 50 °C. It is desirable to carry out the process b) under normal pressure, although a higher or lower pressure may also be used.

In carrying out the reaction of the above-mentioned process b), use is made, for example, of the compounds of the formula (IV) in a mole amount from 1.0 to 1.2 moles per one mole of the compounds of the formula (Ia) in the presence of an inert solvent such as, for example, acetonitrile to obtain the desired compounds of the formula (I).

The active compounds according to the invention exhibit a powerful microbicidal action and can be employed in practice for combating undesired microorganisms. The active compounds are suitable for use as plant protection agents.

Fungicidal agents in plant protection are employed for combating Plasmodiophoromycetes, Oomycetes,

Chytridiomycetes, Zygomycetes, Ascomycetes, Basidiomycetes and Deuteromycetes.

Bactericidal agents are employed in plant protection for combating Pseudomonoadaceae, Rhizobiaceae, Enterobacteriaceae, Corynebacteriaceae and Streptomycetaceae.

Some causative organisms of fungal and bacterial diseases included under the abovementioned main headings, are mentioned below as non-limiting examples:

Xanthomonas species, such as, for example, Xanthomonas campestris pv. oryzae;
Pseudomonas species, such as, for example, Pseudomonas syringae pv. lachrymans;
Erwinia species, such as, for example, Erwinia amylovora;
Pythium species, such as, for example, Pythium ultimum;
Phytophthora species, such as, for example, Phytophthora infestans;
Pseudoperonospora species, such as, for example, Pseudoperonospora cubensis;
Plasmopara species, such as, for example, Plasmopara viticola;
Peronospora species, such as, for example, Peronospora pisi or P. brassicae;
Erysiphe species, such as, for example, Erysiphe graminis;
Sphaerotheca species, such as, for example, Sphaerotheca fuliginea;
Podosphaera species, such as, for example, Podosphaera leucotricha;
Venturia species, such as, for example, Venturia inaequalis;
Pyrenophora species, such as, for example, Pyrenophora teres or P. graminea;
(Conidial form: Drechslera, Synonym: Helminthosporium);
Cochliobolus species, such as, for example, Cochliobolus sativus;
(Conidial form: Drechslera, Synonym: Helminthosporium);
Uromyces species, such as, for example, Uromyces appendiculatus;
Puccinia species, such as, for example, Puccinia recondita;
Tilletia species, such as, for example, Tilletia caries;
Ustilago species, such as, for example, Ustilago nuda or Ustilago avenae;
Pellicularia species, such as, for example, Pellicularia sasakii;
Pyricularia species, such as, for example, Pyricularia oryzae;
Fusarium species, such as, for example, Fusarium culmorum;
Botrytis species, such as, for example, Botrytis cinerea;
Septoria species, such as, for example, Septoria nodorum;
Leptosphaeria species, such as, for example, Leptosphaeria nodorum;
Cercospora species, such as, for example, Cercospora canescens;
Alternaria species, such as, for example, Alternaria brassicae;
Pseudocercosporella species, such as, for example, Pseudocercosporella herpotrichoides.

The good toleration, by plants, of the active compounds, at the concentrations required for combating plant diseases, permits treatment of above-ground parts of plants, of vegetative propagation stock and seeds, and of the soil.

The active compounds can be converted into the customary formulations, such as solutions, emulsions, wettable powders, suspensions, powders, foams, pastes, granules, aerosols, natural and synthetic materials impregnated with active compound, very fine capsules in polymeric substances, coating compositions for use on seed, and formulations used with burning equipment, such as fumigating cartridges, fumigating cans and fumigating coils, as well as ULV cold mist and warm mist formulations.

These formulations may be produced in known manner, for example by mixing the active compounds with extenders, that is to say liquid or liquefied gaseous or solid diluents or carriers, optionally with the use of surface-active agents, that is to say emulsifying agents and/or dispersing agents and/or foam-forming agents. In the case of the use of water as an extender, organic solvents can, for example, also be used as auxiliary solvents.

As liquid solvents diluents or carriers, there are suitable in the main, aromatic hydrocarbons, such as xylene, toluene or alkyl napthalenes, chlorinated aromatic or chlorinated aliphatic hydrocarbons, such as chlorobenzenes, chloroethylenes or methylene chloride, aliphatic hydrocarbons, such as cyclohexane or paraffins, for example mineral oil fractions, alcohols, such as butanol or glycol as well as their ethers and esters, ketones, such as acetone, methyl ethyl ketone, methyl isobutyl ketone or cyclohexanone, or strongly polar solvents, such as dimethylformamide and dimethyl-sulphoxide, as well as water.

By liquefied gaseous diluents or carriers are meant liquids which would be gaseous at normal temperature and under normal pressure, for example aerosol propellants, such as halogenated hydrocarbons as well as butane, propane, nitrogen and carbon dioxide.

As solid carriers there may be used ground natural minerals, such as kaolins, clays, talc, chalk, quartz, attapulgite, montmorillonite or diatomaceous earth, and ground synthetic minerals, such as highly-dispersed

silicic acid, alumina and silicates. As solid carriers for granules there may be used crushed and fractionated natural rocks such as calcite, marble, pumice, sepiolite and dolomite, as well as synthetic granules of inorganic and organic meals, and granules of organic material such as sawdust, coconut shells, maize cobs and tobacco stalks.

As emulsifying and/or foam-forming agents there may be used non-ionic and anionic emulsifiers, such as polyoxyethylene-fatty acid esters, polyoxyethylene-fatty alcohol ethers, for example alkylaryl polyglycol ethers, alkyl sulphonates, alkyl sulphates, aryl sulphonates as well as albumin hydrolysis products. Dispersing agents include, for example, lignin sulphite waste liquors and methylcellulose.

Adhesives such as carboxymethylcellulose and natural and synthetic polymers in the form of powders, granules or latices, such as gum arabic, polyvinyl alcohol and polyvinyl acetate, can be used in the formulation.

It is possible to use colorants such as inorganic pigments, for example iron oxide, titanium oxide and Prussian Blue, and organic dyestuffs, such as alizarin dyestuffs, azo dyestuffs or metal phthalocyanine dyestuffs, and trace nutrients, such as salts of iron, manganese boron, copper, cobalt, molybdenum and zinc.

The formulations in general contain from 0.1 to 95 per cent by weight of active compound, preferably from 0.5 to 90 per cent by weight.

The active compounds according to the invention can be present in the formulations or in the various use forms as a mixture with other known active compounds, such as fungicides, bactericides, insecticides, acaricides, nematicides, herbicides, bird repellents, growth factors, plant nutrients and agents for improving soil structure.

The active compounds can be used as such or in the form of their formulations or the use forms prepared therefrom by further dilution, such as ready-to-use solutions, emulsions, suspensions, powders, pastes and granules. They are used in the customary manner, for example by watering, immersion, spraying, atomising, misting, vaporising, injecting, forming a slurry, brushing on, dusting, scatterting, dry dressing, moist dressing, wet dressing, slurry dressing or encrusting.

In the treatment of parts of plants, the active compound concentrations in the use forms can be varied within a substantial range. They are, in general, from 1 to 0.0001% by weight, preferably from 0.5 and 0.001%.

For the treatment of seed, amounts of active compound of 0.001 to 50 g, especially 0.01 to 10 g, are generally employed per kilogram of seed.

For the treatment of soil, active compound concentrations, at the point of action, of 0.00001 to 0.1% by weight, especially of 0.0001 to 0.02%, are generally employed.

The preparation and use of the active compounds according to the invention can be seen from the following examples.

Preparative Example:

<u>Example 1</u>

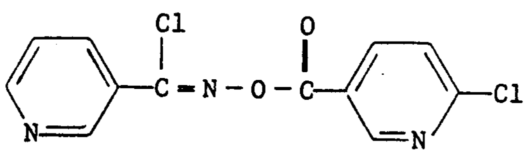

(compound 23)

Triethylamine (5.8 g) was dropwise added to a suspension of 3-(α-chloro-formaldoxime)-pyridinium hydrochloride (5.0 g) in methylene chloride (100 ml) at 0 °C. The reaction mixture was stirred for 30 minutes, and admixed with a solution of 6-chloro-nicotinoyl chloride (4.6 g) in methylene chloride (20 ml). After the temperature of the reaction mixture had been gradually raised to room temperature, the mixture was stirred for 3 hours. Water (100 ml) was added to the reaction mixture, which was then extracted with methylene chloride, dried and distilled to remove the solvent therefrom, so that the desired product i.e. O-6-chloro-nicotinoyl-3-(α-chloro-formaldoxime)-pyridine (7.0 g) was obtained. mp.: 161 - 165 °C.

<u>Example 2</u>

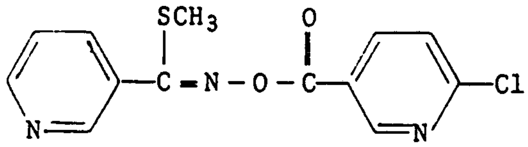

(compound 73)

A mixture of O-6-chloro-nicotinoyl-3-(α-chloro-formaldoxime)-pyridine (5.0 g), a 15% aqueous sodium methyl mercaptan solution (9.5 g) and acetonitrile (50 ml) was stirred at a temperature of 40 to 50 ° C for 3 hours. The liquid reaction mixture was cooled to separate a crystalline material, which was then dried to obtain the desired product i.e. O-6-chloro-nicotinoyl-3-(α-methylthio-formaldoxime)-pyridine (4.2 g). mp.: 129 - 133 ° C.

Example 3

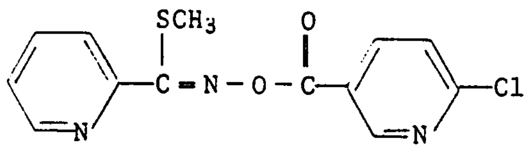

(compound 68)

A mixture of 2-(α-methylthio-formaldoxime)-pyridine (5.0 g), 6-chloro-nicotinoyl chloride (5.2 g), triethylamine (3.3 g) and acetonitrile (50 ml) was stirred for 4 hours, and then distilled under a reduced pressure to remove the solvent therefrom. The resulting residue was admixed with water (100 ml) to crystallize out a material, which was then dried, so that the desired product i.e. O-6-chloro-nicotinoyl-2-(α-methylthio-formaldoxime)-pyridine (7.8 g) was obtained. mp.: 160 - 161 ° C.

The compounds which can be obtained in the same way as Examples 1 to 3 are shown in Table 1, including the compounds obtained in Examples 1 to 3.

Table 1

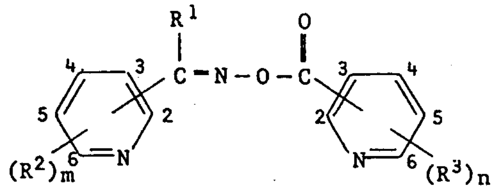

| Comp. No. | $R^1$ | $(R^2)_m$ | Position of 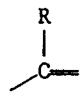 | $(R^3)_n$ | Position of 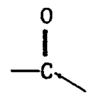 | mp. (°C) |
|---|---|---|---|---|---|---|
| 1 | Cl | - | 2 | - | 2 | 126 - 130 |
| 2 | Cl | - | 2 | - | 3 | 121 - 125 |
| 3 | Cl | - | 2 | - | 4 | 156 - 160 |
| 4 | Br | - | 2 | - | 3 | |
| 5 | F | - | 2 | - | 3 | |
| 6 | Cl | - | 3 | - | 2 | 118 - 122 |
| 7 | Cl | - | 3 | - | 3 | 117 - 119 |
| 8 | Cl | - | 3 | - | 4 | 111 - 115 |
| 9 | I | - | 3 | - | 2 | |
| 10 | Br | - | 3 | - | 3 | |
| 11 | F | - | 3 | - | 3 | |
| 12 | Cl | - | 4 | - | 2 | 131 - 135 |
| 13 | Cl | - | 4 | - | 3 | 131 - 135 |
| 14 | Cl | - | 4 | - | 4 | 136 - 140 |
| 15 | Br | - | 4 | - | 3 | |
| 16 | F | - | 4 | - | 3 | |
| 17 | Cl | - | 3 | 6-$OCH_3$ | 3 | |

| Comp. No. | $R^1$ | $(R^2)_m$ | Position of 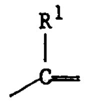 | $(R^3)_n$ | Position of 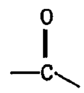 | mp. (°C) |
|---|---|---|---|---|---|---|
| 18 | Cl | - | 3 | 6-Cl | 4 | Viscous solid |
| 19 | Cl | - | 3 | 5-Br | 3 | 173 - 175 |
| 20 | Cl | - | 3 | 6-Cl | 2 | |
| 21 | Cl | - | 3 | 6-$N(CH_3)_2$ | 3 | |
| 22 | Cl | - | 3 | 6-F | 3 | 130 - 132 |
| 23 | Cl | - | 3 | 6-Cl | 3 | 161 - 165 |
| 24 | Br | - | 3 | 6-Cl | 3 | |
| 25 | Cl | - | 3 | 6-Br | 3 | |
| 26 | Cl | - | 3 | 6-$CH_3$ | 2 | |
| 27 | Cl | - | 3 | 2-Cl | 3 | 97 - 101 |
| 28 | Cl | - | 3 | 6-$CF_3$ | 3 | |
| 29 | Cl | - | 3 | 2-$SCH_3$ | 3 | 97 - 101 |
| 30 | Cl | - | 3 | 5-Cl | 3 | 157 - 160 |
| 31 | F | - | 3 | 5-Cl | 3 | |
| 32 | Cl | - | 3 | 5,6-$(CH_3)_2$ | 3 | |
| 33 | Cl | - | 3 | 5,6-$Cl_2$ | 3 | 156 - 160 |
| 34 | Cl | - | 3 | 2,6-$Cl_2$ | 4 | 135 - 138 |
| 35 | Cl | - | 4 | 6-Cl | 3 | 186 - 190 |
| 36 | F | - | 4 | 6-Cl | 3 | |
| 37 | F | - | 4 | 6-F | 3 | |
| 38 | F | - | 4 | 6-$CH_3$ | 3 | |
| 39 | F | - | 4 | 5-Br | 3 | |
| 40 | F | - | 4 | 6-Cl | 2 | |

| Comp. No. | $R^1$ | $(R^2)_m$ | Position of 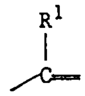 | $(R^3)_n$ | Position of 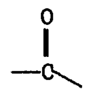 | mp. (°C) |
|---|---|---|---|---|---|---|
| 41 | F | - | 4 | 5,6-$Cl_2$ | 3 | |
| 42 | F | - | 4 | 2-Cl | 4 | |
| 43 | Cl | - | 2 | 6-Br | 3 | |
| 44 | Cl | - | 2 | 5,6-$Cl_2$ | 3 | |
| 45 | Cl | - | 2 | 2,6-$Cl_2$ | 4 | 150 - 153 |
| 46 | Cl | - | 2 | 5,6-$(CH_3)_2$ | 3 | |
| 47 | Br | - | 2 | 4-F | 2 | |
| 48 | Cl | - | 2 | 4-$CH_3$ | 2 | |
| 49 | Cl | - | 2 | 6-Cl | 2 | |
| 50 | Cl | - | 2 | 4-Cl | 2 | |
| 51 | Cl | - | 2 | 6-Cl | 3 | 146 - 150 |
| 52 | Br | - | 2 | 6-Cl | 3 | |
| 53 | Cl | - | 2 | 2-Cl | 3 | 126 - 130 |
| 54 | Cl | - | 2 | 6-F | 3 | 147 - 151 |
| 55 | Cl | - | 2 | 5-Cl | 3 | 121 - 125 |
| 56 | Cl | - | 2 | 5-Br | 3 | Viscous solid |
| 57 | Cl | - | 2 | 6-$OCH_3$ | 3 | |
| 58 | Cl | - | 2 | 6-$CH_3$ | 3 | |
| 59 | Cl | - | 2 | 6-$SCH_3$ | 3 | |
| 60 | Cl | - | 2 | 6-$NHCH_3$ | 3 | |
| 61 | Cl | - | 2 | 6-$N(CH_3)_2$ | 3 | |
| 62 | Cl | - | 2 | 6-$CF_3$ | 3 | |
| 63 | Br | - | 2 | 6-Cl | 3 | |

| Comp. No. | $R^1$ | $(R^2)_m$ | Position of 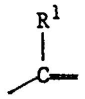 | $(R^3)_n$ | Position of 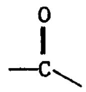 | mp. (°C) |
|---|---|---|---|---|---|---|
| 64 | Br | - | 2 | 5-Br | 3 | |
| 65 | F | - | 2 | 5-Br | 3 | |
| 66 | Cl | - | 2 | 2-$CH_3$ | 3 | |
| 67 | $SCH_3$ | - | 2 | - | 3 | |
| 68 | $SCH_3$ | - | 2 | 6-Cl | 3 | 160 - 161 |
| 69 | $SCH_3$ | - | 2 | 5-Br | 3 | |
| 70 | $SCH_3$ | - | 3 | - | 2 | |
| 71 | $SCH_3$ | - | 3 | - | 3 | |
| 72 | $SCH_3$ | - | 3 | 6-$CH_3$ | 3 | |
| 73 | $SCH_3$ | - | 3 | 6-Cl | 3 | 129 - 133 |
| 74 | $SC_4H_9$-tert | - | 3 | 6-Cl | 3 | 136 - 140 |
| 75 | $SC_2H_5$ | - | 3 | 6-Cl | 2 | |
| 76 | $SCH_3$ | - | 4 | 6-Cl | 3 | |
| 77 | $SCH_3$ | - | 4 | 5-Br | 3 | |
| 78 | $SC_3H_7$-n | - | 4 | 5-Cl | 3 | |
| 79 | Cl | 6-$CH_3$ | 2 | - | 3 | |
| 80 | Cl | 6-$CH_3$ | 2 | 6-Cl | 3 | 163 - 167 |
| 81 | Cl | 4-$CH_3$ | 2 | 6-Cl | 3 | |
| 82 | Cl | 6-$CH_3$ | 3 | 5-Br | 3 | |
| 83 | Cl | 6-Cl | 3 | - | 2 | |
| 84 | Cl | 6-Cl | 3 | - | 3 | 126 - 130 |
| 85 | Cl | 6-Cl | 3 | 6-$OCH_3$ | 3 | |
| 86 | Cl | 6-Cl | 3 | 6-Cl | 3 | 207 - 210 |

| Comp. No. | $R^1$ | $(R^2)_m$ | Position of 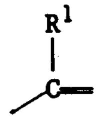 | $(R^3)_n$ | Position of 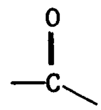 | mp. (°C) |
|---|---|---|---|---|---|---|
| 87 | Cl | 6-Cl | 3 | 2-Cl | 3 | 155 - 158 |
| 88 | Cl | 6-Cl | 3 | 5-Br | 3 | |
| 89 | F | 6-Cl | 3 | 6-Cl | 3 | |
| 90 | Cl | 6-Cl | 3 | 6-Cl | 3 | |
| 91 | Cl | 6-Cl | 3 | 6-$SCH_3$ | 3 | |

Biotest Examples:

Example 4

Control of late blight of tomato

Formulation of active compound:

| | |
|---|---|
| Active compound: | 30 parts |
| Organic solvent (xylene): | 55 parts |
| Emulsifiers: | 8 parts of polyoxyethylene alkylphenyl ether, and 7 parts of calcium alkylbenzenesulfonate |

The above-mentioned components were mixed with one another to form a concentrated emulsion, which was then diluted with water to form a suitable formulation containing a predetermined amount of the active compound.

Test Procedures:

Tomato plants (Kurihara Variety) were grown in porcelain pots each having a size of 9 cm. The emulsion formulation, containing the active compound, was sprayed onto the plants by means of a spray gun.

One day after the treatment, the plants were inoculated with spore suspension of the disease-causing fungi, and kept overnight in a inoculation chamber at a constant temperature of 22°C and a relative humidity of 100%.

After 5 days, the infection rate of the tomato plants was determined and recorded according to the following assessment scale, on the basis of measurement of area wherein the diseased spots had occurred.

| Infection degree | Area wherein the diseased spots occurred (in %) |
|---|---|
| 0 | 0 |
| 0.5 | less than 2 |
| 1 | 2 - less than 5 |
| 2 | 5 - less than 15 |
| 3 | 15 - less than 30 |
| 4 | 30 - less than 50 |
| 5 | 50 or more |

The percent control value was calculated out by employing the formula:

$$\text{Control value (\%)} = \frac{A - B}{A} \times 100$$

wherein A represents the infection degree in the untreated area; and
B represents the infection degree in the treated area.
The results of this test are given in Table 2.

Table 2

| Comp. No. | Concentration of active component (ppm) | Control value (%) |
|---|---|---|
| 18 | 500 | 100 |
| 19 | 500 | 100 |
| 30 | 500 | 100 |
| 45 | 500 | 100 |
| 51 | 500 | 100 |
| 54 | 500 | 100 |

Example 5

Control of anthracnose of cucumber

Test procedures:

Cucumber plants ( Sūyō Variety ) were grown in porcelain pots each having a size of 9 cm.

A diluted emulsion formulation, containing the test compound, was prepared in the manner shown in Example 4, and sprayed on the plants in an amount of 25 ml per three pots.

One day after the above-mentioned treatment, the plants were inoculated, by spraying, with a spore suspension of the disease-causing fungi, and then placed in a chamber at a constant temperature of 23 °C and a relative humidity of at least 90% for one day.

Six days after the inoculation, the infection degree of the plants was determined and recorded according to the following assessment scale.

| Infection degree | Area wherein the diseased spots occurred (in %) |
|---|---|
| 0 | 0 |
| 0.5 | less than 2 |
| 1 | 2 - less than 5 |
| 2 | 5 - less than 15 |
| 3 | 15 - less than 30 |
| 4 | 30 - less than 50 |
| 5 | 50 and higher |

The percent control value was calculated out by employing the formula:

$$\text{Control value (\%)} = \frac{A - B}{A} \times 100$$

wherein A represents the infection degree in the untreated area; and
B represents the infection degree in the treated area.
The results of this test are given in Table 3.

Table 3

| Comp. No. | Concentration of active component (ppm) | Control value (%) |
|---|---|---|
| 35 | 500 | 100 |
| 51 | 500 | 100 |

Example 6

Control of Helminthosporium leaf spots by application of active compounds onto stems and leaves of rice plants

Rice plants (Kusabue Variety) were grown in porcelain pots each having a diameter of 12 cm. The rice plants, at the 3 - 4th leaf stage, were sprayed with 50 ml per 3 pots of a diluted formulation prepared in the manner shown in Example 4 and containing a predetermined amount of the active compound. On the next day, the plants were sprayed two times with spore suspension of Helminthosporium fungi (which had been cultured on PDA medium), and placed in a inoculation chamber at a temperature of 25° C and a relative humidity of 100% for the purpose of infection.

Seven days after the inoculation, the infection degree of the plants per pot was determined and recorded according to the following assessment scale.

| Scale | Infection degree |
|---|---|
| 0 | Not infected |
| 1 | Very slightly infected |
| 2 | Slightly infected |
| 3 | Moderately infected |
| 4 | Considerably infected |
| 5 | Severely infected |

The percent control value was calculated out by employing the formula:

$$\text{Control value (\%)} = \frac{A - B}{A} \times 100$$

wherein A represents the infection degree in the untreated area; and
B represents the infection degree in the treated area.

The results of this test are given in Table 4.

Table 4

| Comp. No. | Concentration of active component (ppm) | Control value (%) |
|---|---|---|
| 19 | 500 | 100 |
| 22 | 500 | 100 |
| 80 | 500 | 100 |

In the tests shown in Examples 4, 5 and 6, it was observed that there were no phytotoxic effects on the test plants.

## Claims

**1.** O-Acyl formaldoxime-pyridines of the formula (I)

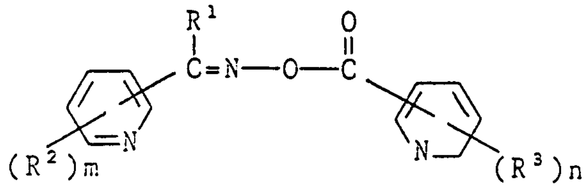

(I)

wherein
$R^1$ is halogen or $C_{1-4}$ alkylthio,
$R^2$ is halogen or $C_{1-4}$ alkyl,
$R^3$ is halogen, $C_{1-4}$ alkyl, $C_{1-4}$ alkoxy,
$C_{1-4}$ alkylthio, $C_{1-2}$ alkylamino,
$C_{2-4}$ (in total) dialkylamino or halo-$C_{1-2}$ alkyl,
m is 0 or 1, and
n is 0, 1 or 2.

**2.** Compounds of the formula (I) according to claim 1
wherein
$R^1$ is fluorine, chlorine, bromine or methylthio,
$R^2$ is chlorine or methyl,
$R^3$ is fluorine, chlorine, bromine, methyl, methoxy, methylthio,
methylamino, dimethylamino or trifluoromethyl,
m is 0 or 1, and
n is 0, 1 or 2.

**3.** Compounds of the formula (I) according to claim 1
wherein
$R^1$ is chlorine or methylthio,
$R^2$ is chlorine or methyl,
$R^3$ is fluorine, chlorine, bromine, methyl, methoxy, methylthio or trifluoromethyl,
m is 0 or 1, and
n is 0, 1 or 2.

**4.** Process for the preparation of the compounds of the formula (I)

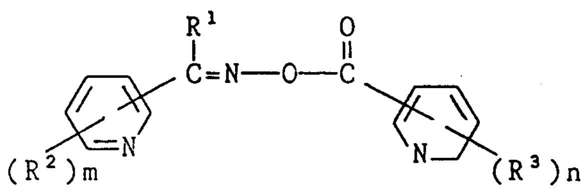

(I)

wherein
$R^1$ is halogen or $C_{1-4}$ alkylthio,
$R^2$ is halogen or $C_{1-4}$ alkyl,
$R^3$ is halogen, $C_{1-4}$ alkyl, $C_{1-4}$ alkoxy,
$C_{1-4}$ alkylthio, $C_{1-2}$ alkylamino,
$C_{2-4}$ (in total) dialkylamino or halo-$C_{1-2}$ alkyl,
m is 0 or 1, and
n is 0, 1 or 2,
characterized in that

a) compounds of the formula (II)

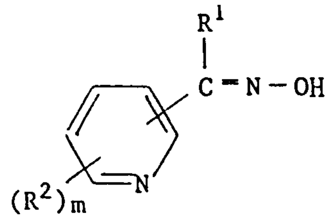

(II)

wherein $R^1$, $R^2$ and m have the same meanings as mentioned above, are reacted with compounds of the formula (III)

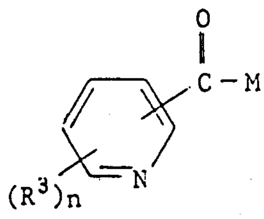

(III)

wherein $R^3$ and n have the same meanings as mentioned above, and
M represents halogen atom,
in the presence of inert solvents, and if appropriate in the presence of an acid binder,
or
b) where $R^1$ represents $C_{1-4}$ alkylthio,
compounds of the formula (Ia)

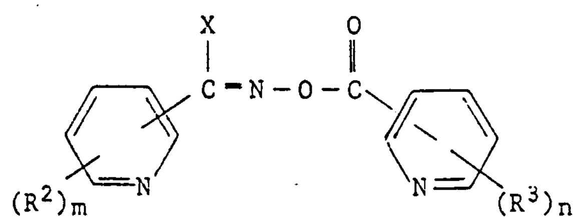

(Ia)

wherein $R^2$, $R^3$, m and n have the same meanings as mentioned above, and
X represents halogen atom,
are reacted with alkylmercaptans of the formula (IV)

alkyl SH (IV)

or their alkali salts
in the presence of inert solvents and if appropriate in the presence of a base.

**5.** Microbicidal compositions, characterized in that they contain at least one O-acyl formaldoxime-pyridine of the formula (I).

**6.** Process for combating microbes, characterized in that O-acyl formaldoxime-pyridines of the formula (I) are allowed to act on microbes and/or their habitat.

**7.** Use of O-acyl formaldoxime-pyridines of the formula (I) for combating microbes.

**8.** Process for the preparation of microbicidal compositions, characterized in that O-acyl formaldoxime-pyridines of the formula (I) are mixed with extenders and/or surface active agents.

European
Patent Office

# EUROPEAN SEARCH REPORT

Application Number

**EP 91 11 5432**

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| D,X | US-A-4 244 959 (F.J. FREENOR, III)<br>* column 3, lines 7 - 28 * * tables A and C; table I; claims 1, 3, 4, 6, 7, 10, 13 *<br>– – – – – | 1-7 | C 07 D 213/78<br>C 07 D 213/83<br>A 01 N 47/40 |

**TECHNICAL FIELDS SEARCHED (Int. Cl.5)**

C 07 D 213/00

The present search report has been drawn up for all claims

| Place of search | Date of completion of search | Examiner |
|---|---|---|
| Berlin | 17 December 91 | HASS C V F |

**CATEGORY OF CITED DOCUMENTS**

X : particularly relevant if taken alone
Y : particularly relevant if combined with another document of the same catagory
A : technological background
O : non-written disclosure
P : intermediate document
T : theory or principle underlying the invention

E : earlier patent document, but published on, or after the filing date
D : document cited in the application
L : document cited for other reasons

& : member of the same patent family, corresponding document